(19) **Europäisches Patentamt / European Patent Office / Office européen des brevets**

(11) **EP 1 586 348 B1**

(12) **EUROPÄISCHE PATENTSCHRIFT**

(45) Veröffentlichungstag und Bekanntmachung des Hinweises auf die Patenterteilung:
**16.04.2008 Patentblatt 2008/16**

(51) Int Cl.:
*A61N 1/365* (2006.01)  *A61N 1/362* (2006.01)

(21) Anmeldenummer: **05090083.6**

(22) Anmeldetag: **31.03.2005**

(54) **Elektrotherapiegerät**

Electrotherapeutic apparatus

Dispositif d'électrothérapie

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **14.04.2004 DE 102004018783**
**09.07.2004 DE 102004034337**

(43) Veröffentlichungstag der Anmeldung:
**19.10.2005 Patentblatt 2005/42**

(73) Patentinhaber: **BIOTRONIK CRM Patent AG**
**6340 Baar (CH)**

(72) Erfinder:
• **Lippert, Michael, Dr.**
**91522 Ansbach (DE)**

• **Czygan, Gerald, Dr.-Ing.**
**91054 Buckenhof (DE)**

(74) Vertreter: **Lindner-Vogt, Karin L.**
**BIOTRONIK GmbH & Co. KG**
**Corporate Intellectual Properties**
**Woermannkehre 1**
**12359 Berlin (DE)**

(56) Entgegenhaltungen:
**EP-A- 0 447 024  EP-A- 0 793 976**
**EP-A- 1 062 974  US-A- 4 773 401**
**US-A- 5 800 467  US-A- 6 154 674**
**US-A1- 2004 138 718**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft ein implantierbares Elektrotherapiegerät, wie beispielsweise einen Herzschrittmacher, einen Defibrillator oder dergleichen. Das Elektrotherapiegerät besitzt einen Aktivitätssensor zum Erfassen einer körperlichen Aktivität und Erzeugen eines entsprechenden Aktivitätspegelsignals. Ein Gehäuse des Elektrotherapiegerätes ist mit einem Elektrodenleitungsanschluss für den Anschluss wenigstens einer intrakardial zu platzierenden Elektrodenleitung versehen. Das Gehäuse selbst oder ein Teil des Gehäuses kann als Neutralelektrode oder Referenzelektrode dienen. Der Elektrodenleitungsanschluss dient dem Anschluss an der Elektrodenleitung, die wenigstens eine Messelektrode für die Aufnahme eines intrakardialen Impedanz- oder Leitfähigkeitssignals besitzt. In dem Gehäuse ist eine Impedanz- oder Leitfähigkeitsmesseinheit angeordnet, die ausgebildet ist, ein uni- oder bipolares Impedanz- oder Leitfähigkeitsverlaufssignal zu erzeugen. Dazu werden während wenigstens eines Herzzyklusses mehrere Impedanz- oder Leitfähigkeitswerte oder ein entsprechender Impedanz- oder Leitfähigkeitsverlauf gemessen. Dies geschieht entweder unipolar durch Messen zwischen einer Neutralelektrode und einer Messelektrode oder zwischen zwei Messelektroden. Außerdem ist in dem Gehäuse eine Auswerteeinheit angeordnet, die zum Auswerten des Impedanz- oder Leitfähigkeitsverlaufs und zum Ableiten eines Kontraktilitätswertes aus dem Impedanz- oder Leitfähigkeitsverlauf dient. Elektrotherapiegeräte, die die Kontraktilität eines Herzens ermitteln können, bieten die Möglichkeit, einer von dem Elektrotherapiegerät abzugebene Therapie an den jeweiligen Kontraktilitätszustand des Herzens des Patienten anzupassen.

Die Kontraktilität beschreibt einen inotropen Zustand eines Herzens. Sie beeinflusst die Kraft und die Geschwindigkeit einer myokardialen Kontraktion. Die Kontraktilität wird durch drei Mechanismen gesteuert:

eine direkte Steuerung durch das autonome Nervensystem (ANS),

den sogenannten Starling-Mechanismus, und

den sogenannten Bowditch-Effekt (Kraft-Frequenzkopplung).

Der Hauptmechanismus, die Steuerung der Kreislaufregulation durch das autonome Nervensystem, vergrößert die Kontraktilität und die Herzrate, wenn ein erhöhter metabolischer Bedarf vorliegt, beispielsweise bei körperlicher oder physischer Anstrengung, um eine geeignete Blutversorgung sicherzustellen.

Bei Patienten mit chronischem Herzversagen (Chronic Heart Failure, HF) nimmt die myokardiale Kontraktilität bis auf ein niedriges Niveau ab und die interventrikuläre Synchronisation wird verschlechtert. Dies wird von einem geringen Auswurfanteil (Ejection Fraction, EF) begleitet, sowie von einer geringen Lebensqualität und einer hohen Sterblichkeit. HF kommt in der Bevölkerung häufig vor. HF-Patienten werden mit verschiedenen Medikamenten behandelt, die den inotropen Zustand beeinflussen, beispielsweise Betablockern, um die Herzrate zu stabilisieren, aber auch mit positiven inotropen Medikamenten, zum Beispiel Glykosiden, um die Kontraktilität zu erhöhen. In jüngerer Zeit werden HF-Patienten mit Resynchronisationstherapie-Geräten, beispielsweise 3-Kammer-Herzschrittmachern oder Defibrillatoren behandelt. Ziel einer solchen Schrittmachertherapie ist es, die zwei Ventrikel eines Herzens durch biventrikuläre Stimulation zu synchronisieren, um das Zeitverhalten der Herzkammern zu verbessern und damit die Herzleistung (Cardiac Resynchronisation Therapy, CRT).

Die Kontraktilität ist daher eine wichtige zu beobachtende Größe, insbesondere für HF-Patienten. Solche Beobachtung ist wichtig, um

den Zustand des Patienten und eine Linderung oder ein Fortschreiten der Krankheit zu beobachten,

eine Resynchronisationstherapie des Herzens (Cardiac Resynchronisation Therapy, CRT) festzulegen und zu beobachten, und

eine Medikamentenbehandlung zu beobachten.

Die Information über die Kontraktilität kann außerdem dazu verwendet werden, eine Herzschrittmachertherapie oder eine Therapie durch einen implantierbaren Kardioverter/Defibrillator (implantable Cardioverter/Defibrillator, ICD) zu optimieren.

Obwohl die Kontraktilität eine Größe von großer Wichtigkeit ist, ist sie in der klinischen Praxis schwer zu messen. Es ist üblich, die Kontraktilität anhand eines maximalen ventrikulären Druckgradienten $dp/dt_{max}$ im rechten Ventrikel oder im linken Ventrikel zu bestimmen. Ein linksventrikulärer Auswurfanteil kann außerdem mittels einer Echokardiographie bestimmt werden. Eine Untersuchung des rechten Ventrikels mittels Echokardiographie ist aus anatomischen Gründen sehr schwierig, obwohl die Information über den rechten Ventrikel für eine vollständige Untersuchung sehr wichtig ist. Beide Vorgehensweisen, die Druckmessung und die Echokardiographie sind zeitaufwendig und teuer. Die ventrikuläre Druckmessung erfordert einen invasiven Eingriff. Sie erfordert einen Druckkatheter in einem oder beiden Ventrikel und kann nur während einer elektrophysiologischen Studie oder der Implantation eines Herzschrittmachers oder Kardioverters/Defibrillators durchgeführt werden.

Implantate, mit denen die Kontraktilität eines Herzens ermittelt werden kann, sind beispielsweise in den US Patenten 4,674,518 und 5,417,717 beschrieben. Dort erfolgt eine Messung der Änderung des Herzkammervolumens mittels des Druckgradientens dP/dt und mittels Impedanz-Plethysmografie.

In der Europäischen Patentanmeldung 1 062 974 ist ein Herzschrittmacher der eingangs genannten Art beschrieben. Das dort beschriebene Steuerverfahren für ein Elektrotherapiegerät und das damit offenbarte Elektrotherapiegerät sollen weiter gebildet werden, um den Einsatzbereich des Elektrotherapiegerätes zu erweitern.

Diese Aufgabe wird durch die in Anspruch 1 angegebene Merkmale gelöst.

Vorzugsweise ist die Auswerteeinheit zum Auswerten des von der Impedanz-oder Leitfähigkeitsmesseinheit erzeugten Impedanz- oder Leitfähigkeitsverlaufssignals und zum Erzeugen und Ausgeben eines Kontraktilitätsdifferenzsignals in Abhängigkeit von dem Impedanz- oder Leitfähigkeitsverlaufssignal durch Bilden einer Differenz zwischen zwei Impedanz- bzw. Leittähigkeitsverlaufssignalen zu unterschiedlichen Zeiträumen derart ausgebildet, dass ein jeweiliger Kontraktilitätsdifferenzwert des Kontraktilitätsdifferenzsignals von einer Fläche abhängt, die zwei zu unterschiedlichen Zeiträumen erfasste Impedanz- oder Leitfähigkeitsverlaufssignale zwischen sich einschließen. Die Auswerteeinheit ist weiterhin dazu ausgebildet, einem jeweiligen auf diese Weise bestimmten Kontraktilitätsdifferenzwert einen Aktivitätssignalwert zuzuordnen, der für wenigstens einen der Zeiträume gilt, in denen die Impedanz- oder Leitfähigkeitsverlaufssignale aufgenommen wurden, die zur Bildung des jeweiligen Kontraktilitätsdifferenzwertes beigetragen haben.

Die von zwei zu unterschiedlichen Zeiträumen erfassten Impedanz- oder Leitfähigkeitsverlaufssignalen eingeschlossene Fläche kann dabei eine gedachte Fläche sein, die sich bei graphischer Darstellung der Impedanzverläufe ergäbe.

Diese Fläche ist im übrigen bereits in der EP 1 062 974 beschrieben, jedoch ohne Bezug auf den dort dargestellten Leitfähigkeitsverläufen zuzuordnende Zeiträume und ohne die entsprechende automatische Zuordnung von Aktivitätswerten. Diese Zuordnung erlaubt es, eine gegebenenfalls vorhandene oder nicht vorhandene Kontraktilitätsdifferenz einer vorhandenen oder nicht vorhandenen Aktivitätsdifferenz zuzuordnen.

Anstelle einer Messung des Impedanzverlaufs kann auch der Verlauf des Kehrwertes der Impedanz, d.h. der Verlauf der Leitfähigkeit gemessen werden, siehe EP 1 062 974.

Wenn ähnlich wie bereits in der EP 1 062 974 beschrieben ein Leitfähigkeits-oder Impedanzverlaufssignal für ein bekanntes Aktivitätssignal (beispielsweise im Ruhezustand des Patienten aufgenommen) gespeichert wird, und dieses gespeicherte Impedanzverlaufssignal regelmäßig zur Differenzbildung mit einem weiteren Impedanzverlaufssignal herangezogen wird, reicht es grundsätzlich, wenn das gespeicherte Aktivitätssignal die Aktivität während des Zeitraums widerspiegelt, zu dem das weitere Impedanzverlaufssignal aufgenommen wurde.

Es ist jedoch vorgesehen, auf eine Speicherung eines einmal vorgegebenen Referenzimpedanzsignalverlaufs ganz zu verzichten. Stattdessen soll ein Referenzsignalverlauf von vornherein in Abhängigkeit von gleichzeitig aufgenommenen Aktivitätspegelsignalwerten gebildet werden. Dies geschieht vorzugsweise, indem nur solche Impedanz- oder Leitfähigkeitssignalverläufe für das Bilden eines gemittelten Referenzsignalverlaufs herangezogen werden, die mit Aktivitätspegelwerten unterhalb eines vorgegebenen Grenzaktivitätspegelwertes einhergehen. Die derart zur Bildung eines Referenzsignalverlaufs herangezogenen Impedanz- oder Leitfähigkeitssignalverläufe werden in jedem Fall zunächst mit dem jeweils zeitlich zugeordneten Aktivitätspegelwert normiert, bevor die derart normierten Impedanz- oder Leitfähigkeitssignalverläufe anschließend zum Bilden des Referenzsignalverlaufs gemittelt werden. Zusätzlich zur Normierung eines Kontraktilitätssignals (berechnet aus Impedanzverlauf) selbst mit der Aktivität kann auch eine Normierung des Kontraktilitätsdifferenzsignals (Differenzfläche zwischen aktuellem und ReferenzImpedanzverlauf) mit der Aktivitätspegelsignalwertdifferenz erfolgen. Damit wird ein auf die Aktivitätsdifferenz normiertes Kontraktilitätsdifferenzsignal erzeugt, das ein Maß für die Fähigkeit des Herzens ist, bei steigender Belastung (gemessen als physische Aktivität) auch die Kontraktilität (gemessen über den Impedanzverlauf) zu erhöhen.

Vorzugsweise haben die Zeiträume, über die die Impedanzverlaufssignale jeweils aufzunehmen sind, die Dauer von wenigstens einem definierten Teil eines Herzzyklusses oder eines ganzen Herzzyklusses oder einer ganzzahligen Anzahl von wenigen Herzzyklen, also nicht 100 Herzzyklen sondern beispielsweise nur 8 oder 16 Herzzyklen.

Das Elektrotherapiegerät ist weiterhin vorzugsweise dazu ausgebildet, ein jeweiliges Impedanzverlaufssignal durch eine unipolare Impedanzmessung zu bilden, bei der die Messung zwischen einer am Gehäuse des Elektrotherapiegerätes angeordneten Neutralelektrode und einer Messelektrode im Herzen erfolgt.

Vorzugsweise besitzt die Impedanz- oder Leitfähigkeitsmesseinheit einen Eingangsverstärker mit einer automatischen Eingangsverstärkungssteuerung (ACG), so dass das Ausgangssignal eines Impedanz- oder Leitfähigkeitssensors jeweils möglichst optimal an einen nachfolgenden Analog-Digital-Wandler angepasst ist. Die Impedanz- oder Leitfähigkeitsmesseinheit ist dabei dazu ausgebildet, das jeweils verstärkte Ausgangssignal des Eingangsverstärkers nach dessen Analog-Digital-Wandlung mit dem dazugehörigen Verstärkungsfaktor zu skalieren.

Eine vorteilhafte Ausführungsvariante eines implantierbaren Elektrotherapiegerätes in Form eines Herzschrittmachers oder Defibrillators ergibt sich, wenn in dem Gehäuse der Therapiegerätes eine Therapiegerätesteuereinheit vorgesehen ist, die mit der Auswerteeinheit verbunden ist und wenigstens einen Therapieparameter wie uni- oder biventrikuläre Stimulation, interventrikuläre Verzögerungszeit, atrio-ventrikuläre Verzögerungszeit in Abhängigkeit eines jeweiligen Kontraktilitätssignals oder eines Kontraktilitätsdifferenzsignals einzustellen. Dabei ist die Therapiegerätesteuereinheit vorzugsweise derart ausgebildet, dass sie den jeweiligen Therapieparameter automatisch so einstellt, dass das Kontraktilitätssignal oder das Kontraktilitätsdifferenzsignal eine maximale Kontraktilität anzeigt.

Vorzugsweise ist in dem Gehäuse ein Datenspeicher für das Kontraktilitätssignal und vorzugsweise zusätzlich für das Aktivitätspegelsignal vorgesehen. Dieser Datenspeicher ist im Folgenden auch als Speicher bezeichnet und erlaubt es, sowohl die für die Berechnung des Kontraktilitätsdifferenzsignals erforderlichen Leitfähigkeits- oder Impedanzsignalverläufe zu speichern als auch den Verlauf des jeweiligen Kontraktilitätsdifferenzsignals selbst, um dieses in einer bevorzugten Ausführungsvariante telemetrisch abrufen zu können.

Der Datenspeicher umfasst vorzugsweise mehrere Speicherbereiche, die zum getrennten Speichern beispielsweise eines Referenzsignalverlaufs und wenigstens eines aktuellen Impedanz- oder Leitfähigkeitssignalverlaufs sowie eines Kontraktilitätssignalverlaufs oder Kontraktilitätsdifferenzsignalverlaufs und des zugeordneten Aktivitätspegelsignalverlaufs dienen. Die hier genannten Speicherbereiche enthalten Daten zu unterschiedlichen Zeitbereichen: Die Daten zum Referenzsignalverlauf und zum Impedanzsiganlverlauf: betreffen typischerweise einen Zeitraum von weniger als einem Herzzyklus, während die Daten zum Kontraktilitätssignalverlauf oder zum Kontraktilitätsdifferenzsignalverlauf und zum Aktivitätspegelsignalverlauf jeweils einen Zeitraum von bis zu mehreren Monaten betreffen.

Der Datenspeicher ist vorzugsweise mit der Therapiesteuereinheit verbunden. Zusätzlich ist der Datenspeicher vorzugsweise mit der Auswerteeinheit verbunden.

In dem Gehäuse ist in einer bevorzugten Ausführungsvariante eine Datentelemetrieeinheit angeordnet, die mit der Therapiesteuereinheit oder dem Datenspeicher oder beiden verbunden ist.

Die Erfindung soll nun anhand eines Ausführungsbeispiels mit Bezug auf die Figuren näher erläutert werden. Von den Figuren zeigen

Figur 1: eine implantierbare Therapievorrichtung in Form eines Herzschrittmachers sowie eine zugehörige Elektrodenleitung und ein Herz in schematischer Darstellung;

Figur 2: eine simulierte Potentialverteilung für eine Anordnung gemäß Figur 1;

Figur 3: Impedanzverlaufssignale, aufgenommen mit der Anordnung aus Figur 1 für einen unbelasteten und einen belasteten Zustand samt Darstellung einer Differenzfläche; und

Figur 4: ein schematisches Blockschaltbild der implantierbaren Therapievorrichtung aus Figur 1.

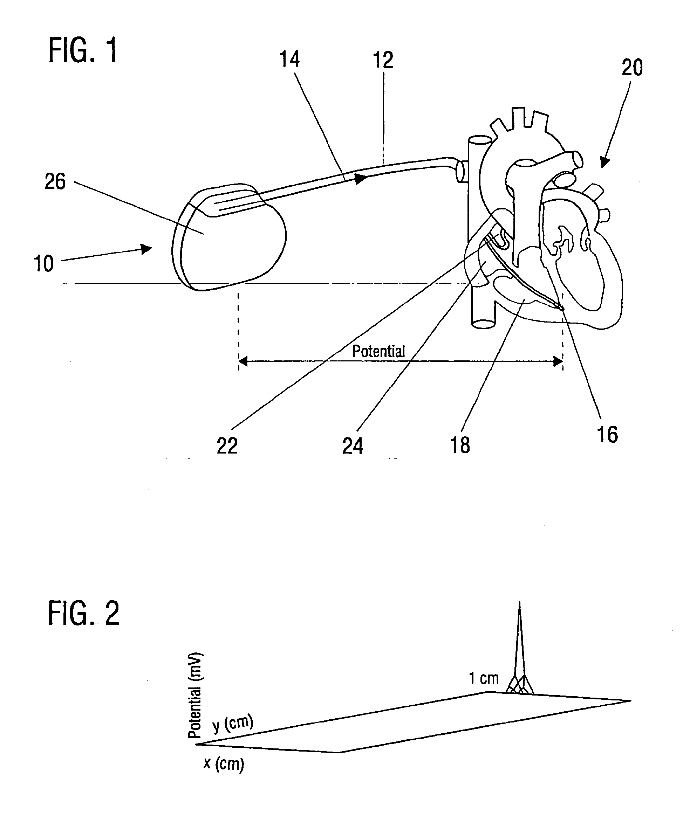

Die in Figur 1 dargestellte Anordnung umfasst einen Herzschrittmacher 10, an den zwei Elektrodenleitungen angeschlossen sind, nämlich eine atriale Elektrodenleitung 12 und eine ventrikuläre Elektrodenleitung 14. Die ventrikuläre Elektrodenleitung 14 besitzt eine Spitzenelektrode 16, die im Apex einer rechten Herzkammer 18 eines schematisch dargestellten Herzens 20 platziert ist. Die atriale Elektrodenleitung 12 ist im Bereich ihres distalen Endes j-förmig gebogen und besitzt eine atriale Spitzenelektrode 22, die im rechten Atrium 24 des Herzen 20 angeordnet ist.

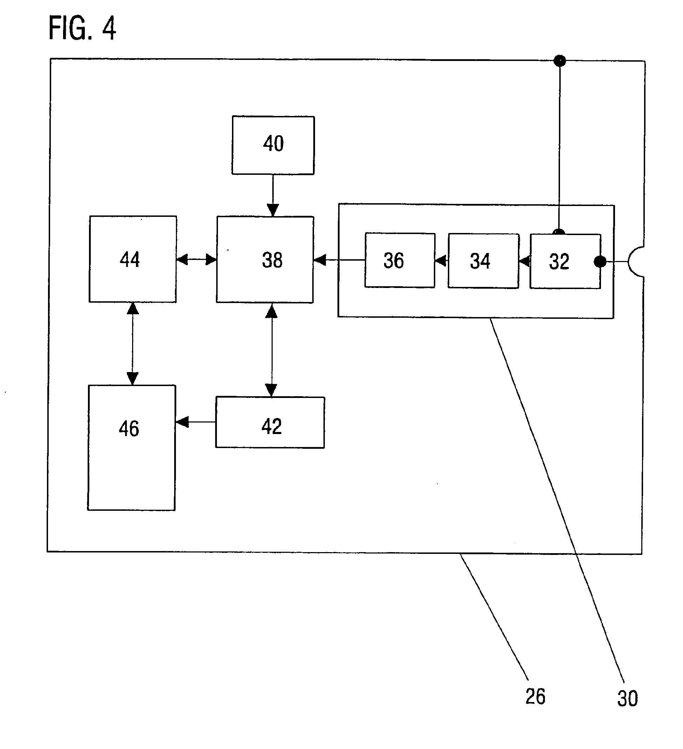

Zur Aufnahme des hier interessierenden Impedanzverlaufs wird die Impedanz zwischen der ventrikulären Spitzenelektrode 16 und einer von einem Gehäuse 26 des Schrittmachers 10 gebildeten Neutralelektrode gemessen. Die Neutralelektrode 26 und die ventrikuläre Spitzenelektrode 16 sind dazu mit einem im Inneren des Gehäuses 26 angeordneten und in Figur 4 schematisch dargestellten Eingangsverstärker 34 verbunden. Dieser Eingangsverstärker 34 besitzt eine automatische Verstärkungssteuerung, die bewirkt, dass ein zwischen der Neutralelektrode 26 und der ventrikulären Spitzenelektrode 16 gemessenes Impedanzsignal mit einem veränderlichen Verstärkungsfaktor verstärkt wird. Die automatische Verstärkungssteuerung (Automatic Gain Control, AGC) bewirkt, dass das Ausgangssignal eines Impedanzsensors möglichst optimal an einen nachgeschalteten Analog-Digital-Wandler 36 angepasst wird. Impedanzsensor 32, Filter mit Eingangsverstärker 34 und Analog-Digital-Wandler 36 sind Bestandteil einer Impedanzmesseinheit 30, die als Ausgangssignal ein mit dem jeweils eingestellten Verstärkungsfaktor skaliertes Impedanzverlaufssignal liefert. Dieses Impedanzverlaufssignal kann ein quasi-kontinuierliches Signal sein oder ein in größeren Zeitabständen zeitabgetastetes, mittels einer Sample- und Hold-Schaltung gesampeltes Impedanzsignal sein. Die automatische Verstärkungssteuerung dient der optimalen Anpassung des Impedanzsensorausgangssignals an den AD-Wandler.

Im Inneren des Gehäuses 26 des Herzschrittmachers 10 ist außerdem ein Aktivitätssensor 40 angeordnet, der geeignet ist, einen jeweiligen Aktivitätspegel eines Patienten zu erfassen und ein entsprechendes Aktivitätspegelsignal zu erzeugen. Der Aktivitätssensor 40 ist in einer bevorzugten Ausführungsform ein Accelerometer (Beschleunigungsmesser) und misst entsprechend die jeweilige Beschleunigung des Herzschrittmachers 10.

Der Impedanzeinheit 30 und dem Aktivitätssensor 40 nachgeschaltet ist eine ebenfalls im Gehäuse 26 angeordnete Auswerteeinheit 38.

Außerdem ist in dem Gehäuse 26 des Herzschrittmachers 10 ein Impedanzverlaufsspeicher 42 angeordnet, der wenigstens mittelbar mit der Auswerteeinheit 38 verbunden ist und direkt mit der Impedanzmesseinheit 30 verbunden sein kann.

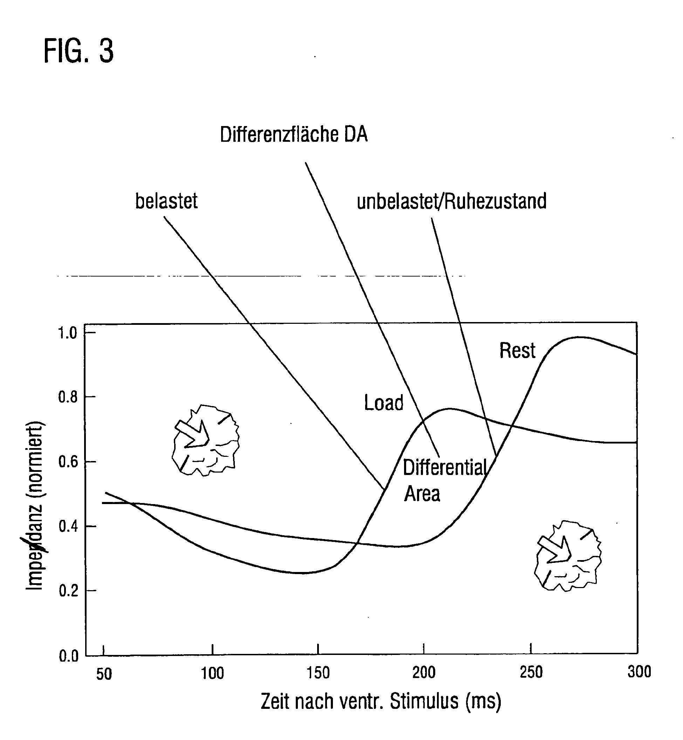

Die Auswerteeinheit 38 ist ausgebildet, ein Kontraktilitäts- beziehungsweise Kontraktilitätsdifferenzsignal aus zwei zu unterschiedlichen, aber gleich langen Zeiträumen erfassten Impedanzverlaufssignalen zu bilden. Dazu wird das Kontraktilitätsdifferenzsignal derart gebildet, dass es in etwa einer zwischen zwei Impedanzverlaufssignalen eingeschlossen Fläche entspricht (siehe Figur 3, Differenzfläche DA). Das Ermitteln dieses Kontraktilitätsdifferenzsignals geschieht vorzugsweise, indem für die beiden Impedanzverlaufssignale zunächst ein Bezugs-Nullzeitpunkt ermittelt wird, beispielsweise gegeben durch eine jeweilige R-Zacke. Anschließend wird für eine Reihe aufeinanderfolgender Zeitabstände vom Bezug-Nullzeitpunkt jeweils der Absolutwert der Differenz der beiden Impedanzverlaufssignale gebildet und schließlich die Summe dieser absoluten Differenzwerte über die unterschiedlichen Zeitabstände gebildet. Auf diese Weise ergibt sich ein Kontraktilitätsdifferenzwert, der der Summe der Absolutwerte der Flächenintegrale der von den beiden Impedanzverlaufssignalen eingeschlossenen Flächen entspricht.

Wie zuvor beschrieben, wird ein jeweiliger Impedanzverlauf unipolar gemessen. Wie Figur 2 zeigt, spiegelt ein derartiger, unipolar im Apex einer Herzkammer gemessener Impedanzverlauf im wesentlichen den Verlauf der lokalen Impedanz um die Messelektrode (ventrikuläre Spitzenelektrode) herum wider.

Die beanspruchte Vorrichtung beruht auf der Beobachtung der ventrikulären Kontraktilität mittels eines implantierten Herzschrittmachers oder Kardioverters/Defibrillators mittels intrakardialer Impedanzmessung. Die in Figur 2 abgebildete Berechnung eines elektromagnetischen Feldes zeigt, dass ein unipolar mittels einer in der Spitze der Herzkammer angeordneten Messelektrode gemessener Impedanzwert Impedanzänderungen in naher Umgebung der Messelektrode widerspiegelt.

Das gemessene Impedanzsignal verändert sich während der Kontraktion des Herzens, weil Blut und das Myokardgewebe unterschiedliche elektrische Leitfähigkeiten besitzen. Daher gibt der Zeitverlauf der unipolar gemessenen Impedanz die Kontraktionsdynamik des ventrikulären Apex wieder. Es ist bekannt, dass das unipolar gemessene rechtsventrikuläre Impedanzsignal gut mit dem Maximum der Druckänderung im rechten Ventrikel während eines Herzzyklusses übereinstimmt.

Die Impedanzmessung selbst ist an sich bekannt. Ein konstanter, gepulster Strom wird zwischen zwei Elektroden eingespeist und die daraus resultierende Spannung wird zeitabgetastet, gefiltert, verstärkt und analog-digital gewandelt.

Grundsätzlich können alle verfügbaren Elektroden als stromeinspeisende Elektroden und als spannungsmessende Elektroden verwendet werden. Dies gilt insbesondere für Anordnungen, bei denen mehr Elektroden vorgesehen sind als bei der in Figur 1 abgebildeten Anordnung. Dabei können dieselben Elektroden sowohl zum Einspeisen des Stroms als auch zum Messen der Spannung verwendet werden. Bei der in Figur 1 dargestellten, bevorzugten Ausführungsvariante wird die Spannung unipolar zwischen Herzschrittmachergehäuse 26 und ventrikulärer Spitzenelektrode 16 gemessen und auch der Messstrom wird zwischen diesen beiden Elektroden eingespeist. Anstelle der rechtsventrikulären Spitzenelektrode kann auch eine beispielsweise im Coronar-Sinus oder einer davon abzweigenden Lateralvene angeordnete, linksventrikuläre Elektrode als Messelektrode dienen. Aus dem so gewonnenen Impedanzverlaufssignal wird das bereits angesprochene Kontraktiliätssignal abgeleitet, und zwar vorzugsweise im Form eines Kontraktilitätsdifferenzsignals, welches durch Vergleich eines Impedanzsignalverlaufs im Ruhezustand des Patienten und eines Impedanzsignalverlaufs im Belastungszustand des Patienten gewonnen wird.

Wie zuvor bereits erwähnt, wird das Kontraktilitätsdifferenzsignal vorzugsweise so gebildet, dass es die Differenzfläche widerspiegelt, die von zwei zu unterschiedlichen Zeitpunkten aufgenommenen Impedanzverlaufssignalen eingeschlossen ist. Impedanzverlaufssignale für einen Ruhezustand und einen Belastungszustand sind in Figur 3 dargestellt. Außerdem ist die von diesen beiden Impedanzverlaufssignalen eingeschlossene Differenzfläche DA dargestellt.

Wie bereits gesagt, ist die Differenzfläche die absolute Differenz zwischen dem Impedanzverlaufssignal für den Ruhezustand des Patienten und dem Impedanzverlaufssignal für den Belastungszustand des Patienten. Die Differenzfläche DA (Differential Area) lässt sich für zwei Impedanzverlaufssignale mit jeweils N Abtastwerten, für die jeweils ein Belastungsimpedanzwert $Z_{bel}$ (oder akuter Impedanzwert $Z_{aku}t$) und ein Ruheimpedanzwert $Z_{Ruhe}$ (oder Referenzimpedanzwert $Z_{Ref}$) vorliegt wie folgt berechnen:

$$ DA = \frac{1}{N} \sum_{i=1}^{N} \left| Z_{akut}(i) - Z_{Ref}(i) \right| $$

Die beiden Impedanzverlaufssignale beziehungsweise deren Abtastindizes i sind dabei auf einen geeigneten Bezugs-Nullzeitpunkt bezogen, beispielsweise jeweils die R-Zacke im zugehörigen Elektrokardiogramm.

Anstelle eines Ruheimpedanzverlaufs mit Ruheimpedanzwerten $Z_{Ruhe}$ und eines Belastungsimpedanzverlaufs mit Impedanzwerten $Z_{bl}$ kann auch ein beliebiger Referenzimpedanzverlauf als Referenzsignal mit Impedanzwerten $Z_{Ref}$ und ein jeweils aktueller Impedanzsignalverlauf mit Impedanzwerten $Z_{akut}$ herangezogen werden.

Die Impedanz wird jeweils kontinuierlich gemessen, beispielsweise zu jedem Herzschlag. Der aktuelle Impedanzverlauf wird als Kurzzeitmittelwert mit einer kleinen Zeitkonstante gebildet, beispielsweise einem 15/16 rekursiven Tiefpassfilter. Der Referenzimpedanzverlauf ist vorzugsweise ein gemittelter Ruheimpedanzsignalverlauf der nur aus solchen Impedanzsignalverläufen gebildet wird, die im Ruhezustand des Patienten aufgenommen worden. Dieser Referenzimpedanzverlauf wird mit einer langen Zeitkonstante gefiltert, beispielsweise mit einem 255/256 rekursiven Tiefpassfilter.

Ob sich ein Patient im Ruhezustand oder im Belastungszustand befindet, wird anhand des Ausgangssignals des Aktivitätssensors, also des Accelerometers bestimmt. Dieses Accelerometer gibt ein Aktivitätspegelsignal aus, welches anzeigt, ob ein Patient ruht oder körperlich tätig ist. Aus dem Aktivitätspegelsignal kann ein Bewegungsflag Mflag (Motional Flag) abgeleitet werden, welches gesetzt wird (Mflag =1), wenn ein Ausgangssignal des Accelerometers einen vorgegebenen Grenzwert überschreitet. Das Bewegungsflag Mflag wird zurückgesetzt (Mflag = 0), wenn das Ausgangssignal des Accelerometers einen vorgegebenen zweiten Grenzwert unterschreitet der erste und der zweite Grenzwert können unterschiedlich, aber auch identisch sein. Das Referenzimpedanzverlaufssignal wird nur dann gebildet, wenn das Bewegungsflag zurückgesetzt ist (Mflag=0).

Das Mflag dient zur Steuerung der Mittelung des Referenz-Impedanzsignalverlaufs. Es soll nicht das Aktivitätspegelsignal ersetzen, da (z.B. zur Normierung des Kontraktilitätsreferenzsignals) das Aktivitätspegelsignal mehrere Abstufungen des Aktivitätspegels wiedergeben können soll.

Wenn im Belastungsfall des Patienten der erste Grenzwert überschritten wird und das Mflag gesetzt ist (Mflag=1 ), wird die Bildung des Referenzimpedanzverlaufssignals unterbrochen. Gleichzeitig wird eine durchschnittliche Differenzfläche $DA_{avg}$ berechnet. Diese durchschnittliche Differenzfläche wird für eine vorgegebene, beispielsweise programmierbare Beobachtungsperiode, beispielsweise 24 Stunden, bestimmt. Mit Beginn jeder neuen Beobachtungsperiode wird eine neue durchschnittliche Differenzfläche $DA_{avg}$ berechnet.

Zusätzlich zur Berechnung der durchschnittlichen Differenzfläche $DA_{avg}$ kann auch ein durchschnittliches Aktivitätspegelsignal ermittelt werden. Jede durchschnittliche Differenzfläche (also jedes durchschnittliche Kontraktilitätsdifferenzsignal) kann dann mit dem jeweils zugehörigen Aktivitätspegelsignalwert normiert werden.

Alternativ kann die durchschnittliche Differenzfläche $DA_{avg}$ (der durchschnittliche Kontraktilitätsdifferenzsignalwert) für verschiedene Ausgangswertebereiche des Accelerometers berechnet werden. Außerdem ist es möglich, einen jeweiligen Kontraktilitätsdifferenzsignalwert (die Differenzfläche) mit einem jeweils zugehörigen Kurzzeit gemittelten Aktivitätspegelsignalwert zu normieren, bevor die gemittelte Differenzfläche, also der gemittelte Kontraktilitätsdifferenzsignalwert gebildet wird. Durch diese Normierung wird sichergestellt, dass Beobachtungsperioden mit unterschiedlichen Aktivitätspegeln vergleichbar sind.

Der zu messende Impedanzverlauf hängt nicht allein von der durch die Kontraktilität beeinflussten Kontraktionsdynamik ab, sondern auch von der Art des ventrikulären Ereignisses, also davon, ob die ventrikuläre Kontraktion eine natürliche Kontraktion ist, oder eine stimulierte. Der Impedanzverlauf (und der Kontraktionsverlauf) können bei stimulierten und bei intrinsischen, natürlichen ventrikulären Kontraktionen unterschiedlich sein. Daher wird die durchschnittliche Differenzfläche $DA_{avg}$ in einer bevorzugten Ausführungsvariante für die beiden unterschiedlichen Arten ventrikulärer Ereignisse - stimuliert und intrinsisch - separat berechnet.

Zu diesem Zweck sind in der bevorzugten Ausführungsvariante die Auswerteeinheit und eine Therapiesteuereinheit 44, die beispielsweise die Abgabe von Stimulationsimpulsen steuert, derart wenigstens mittelbar miteinander verbunden, dass die Therapiesteuereinheit 44 an die Auswerteeinheit 38 ein Signal abgibt, wenn eine ventrikuläre Stimulation erfolgt. Dieses Signal ist in einer besonders bevorzugten Ausführungsvariante ein an sich bekanntes Markersignal zum Kennzeichnen ventrikulärer Stimulation.

Die auf diese Weise bestimmte durchschnittliche Differenzfläche $DA_{avg}$ ist ein Indikator für die durchschnittliche Kontraktilitätsänderung während einer körperlichen Belastung eines einzelnen Patienten. Dieser Wert kann auf die nachfolgend beschriebene Weise für diagnostische und therapeutische Zwecke genutzt werden.

Grundsätzlich ist es auch möglich, ein die Kontraktilität kennzeichnendes Signal auf andere Weise aus dem Impedanzsignal zu gewinnen, so wie dies beispielsweise bei dem eingangs zitierten Stand der Technik geschieht (beispielsweise per Impedanz-Plethysmographie oder durch Bilden der zweiten Ableitung des Impedanzsignals). Die Unterscheidung zwischen dem Ruhezustand und einem Belastungszustand eines Patienten wie auch die Mittelwertbildung über eine vorgegebene Zeitperiode sollen auch in diesen Fällen (Bestimmung der Kontraktilität aus der Impedanz auf anderem Weg als über die Differenzfläche) so erfolgen, wie hier beschrieben.

Vorzugsweise umfasst der Herzschrittmacher 16 eine Therapiesteuereinheit 44, die ausgebildet ist, einen oder mehrere von ihr zu steuernde Stimulationsparameter wie der Stimulationsmodus - biventrikulär, rechtsventrikulär, linksventrikulär, etc. - eine atrio-ventrikuläre Verzögerungszeit oder eine interventrikuläre Verzögerungszeit in Abhängigkeit des jeweiligen Kontraktilitätssignals so einzustellen, dass sich jeweils eine größtmögliche Kontraktilität ergibt. Diese Optimierung der Stimulationsparameter durch Therapiesteuereinheit 44 erfolgt dabei vorzugsweise nach Art einer Regelung in rekursiver Weise, indem ein zunächst vorgegebener Stimulationsparameter (beispielsweise für die atrio-ventrikuläre Verzögerungszeit oder die interventrikuläre Verzögerungszeit) schrittweise verändert wird und immer derjenige Wert des Steuerparameters als Ausgangspunkt für die nächste Veränderung herangezogen wird, für den sich jeweils die höchste Kontraktilität ergeben hat. In diesem Sinne greift die Therapiesteuereinheit 44 auf einen Speicher 42 für das Kontraktilitätssignal zu, welches in der bevorzugten Ausführungsvariante durch den Verlauf der durchschnittlichen Differenzfläche $DA_{avg}$ gegeben ist.

Diese Therapiesteuereinheit 44 kann zusätzlich wie in der zuvor erwähnten Weise mit der Auswerteeinheit 38 derart verbunden sein kann, dass die Auswerteeinheit ein Markersignal empfängt, wenn eine ventrikuläre Stimulation erfolgt.

In einer weiter bevorzugten Ausführungsvariante weist der Speicher 42 für das Kontraktilitätssignal getrennte Speicherbereiche für die Differenzfläche $DA_{avg}$ für beide Arten ventrikulärer Ereignisse und für die Anzahl der Differenzflächenwerte, die zur Bildung der jeweiligen durchschnittlichen Differenzfläche $DA_{avg}$ beigetragen haben, auf. Dieser Speicher ist vorzugsweise mit einer Telemetrieeinheit 46 verbunden, so dass die in dem Speicher 42 gespeicherten Werte telemetrisch durch einen Arzt abgefragt werden können.

Neben Speicherbereichen für die zuletzt genannten Daten in bezug auf das Kontraktilitätssignal (die Differenzfläche) ist vorzugsweise ein weiterer Speicherbereich für die zugehörigen Aktivitätspegelsignalwerte vorgesehen. Die derart durch den Arzt telemetrisch abzufragenden Daten können dann auf einem Bildschirm graphisch dargestellt werden und die Diagnose durch den Arzt unterstützen.

Mit Hilfe der telemetrischen Anbindung des Herzschrittmachers 16 an ein externes Gerät können die gemittelten Kontraktilitäts- und Aktivitätspegelsignale auch im Rahmen eines sogenannten Homemonitoring regelmäßig, beispielsweise täglich, übermittelt werden. In diesem Falle werden die an ein externes Gerät übertragenen Daten von diesem externen Gerät auf an sich bekannte Weise an ein zentrales Service-Center übermittelt und können mit dort zuvor bereits gespeicherten Daten korreliert werden.

Die Therapiesteuereinheit kann darüber hinaus so ausgebildet sein, dass sie von sich aus eine Datenübertragung auslöst, wenn ein besonderes Ereignis, beispielsweise ein Alarm-Zustand, vorliegt. Dieses besondere Ereignis kann beispielsweise eine detektierte Arrhythmie sein. Genauso ist es möglich, dass das besondere Ereignis eine telemetrisch empfangene Anforderung seitens eines Arztes oder des Patienten selbst ist. Im Zusammenhang mit der Überwachung und der Auswertung des Kontraktilitätssignals kann als besonderes, eine telemetrische Verbindung auslösendes Ereignis auch eine signifikante Veränderung des Kontraktilitätssignals, das heißt im bevorzugten Fall des Wertes der Differenzfläche selbst sein.

Die Beobachtung der mittels eines implantierten Elektrotherapiegerätes ermittelten Kontraktilität auf die zuvor beschriebene Weise kann für verschiedene diagnostische oder therapeutische Zwecke genutzt werden. Zu den diagnostischen Möglichkeiten zählen:

- eine generelle Überwachung von Patienten mit Herzversagen (HF-Patienten) mittels der gespeicherten und telemetrisch übertragenen Daten

- Überwachung der HF-Patienten mittels der Homonitoring-Daten und der zuvor erwähnten Alarm-Funktionalität

- Überwachung einer Medikation, beispielsweise wenn Medikamente mit positiver oder negativer inotroper Wirkung verabreicht werden

- Überwachung einer Resynchronisationstherapie; und/oder

- Beobachtung von Patienten, die ein erhöhtes Risiko für plötzliche Kontraktilitätsänderungen zeigen, beispielsweise Patienten mit dem Risiko eines Myokardinfarkts oder einer Ischämie.

Die therapeutischen Optionen sind:

- Anpassung des Stimulationsmodus an detektierte Kontraktilitätsänderungen, beispielsweise Umschalten zwischen rechtsventrikulärer, linksventrikulärer und biventrikulärer Stimulation, und

- Anpassung anderer Stimulations- oder Defibrillationsparameter beispielsweise solcher Zeitparameter wie die bereits erwähnte atrio-ventrikuläre Verzögerungszeit, die biventrikuläre Verzögerungszeit aber auch der Stimulationsrate selbst oder zusäzlich oder alternativ

- Anpassung oder Steuerung einer Medikamententherapie durch den Arzt.

Darüber hinaus kann ein derartiger Herzschrittmacher auch als akuter Sensor verwendet werden, beispielsweise während einer elektrophysiologischen Untersuchung, wenn der Patient permanent implantierte Elektrodenleitungen besitzt. Die Reaktion des Herzens auf eine temporäre Anwendung eines positivinotropen Medikaments, beispielsweise Dobutamin, oder auf eine standardisierte Belastung, kann ebenfalls getestet werden. Verschiedene Untersuchungen an einem Patienten können miteinander verglichen werden und die Änderungen der jeweiligen Belastungsreaktion ausgewertet werden. Diese Anwendung dient der Diagnose von Herzinsuffizienz, der regelmäßigen Beobachtung der Verschlechterung oder Verbesserung von Herzfehlern und zum Testen der allgemeinen Kontraktilität.

**Patentansprüche**

1. Implantierbares Elektrotherapiegerät, insbesondere Herzschrittmacher, Defibrillator oder dergleichen, mit

einem Gehäuse (26), welches

Einen Elektrodenleitungsanschluss für den Anschluss wenigstens einer intrakardial zu platzierenden Elektrodenleitung aufweist, die wenigstens eine Messelektrode für die Aufnahme eines intrakardialen Impedanzsignals aufweist,

wobei in dem Gehäuse

ein Aktivitätssensor (40), der ausgebildet ist, ein Aktivitätspegelsignal zu erzeugen, dessen jeweilige Aktivitätspegelsignalwerte im implantierten Zustand des Therapiegeräts von einer körperlichen Aktivität eines Patienten abhängen,

eine Impedanz- oder Leitfähigkeitsmesseinheit (30), die ausgebildet ist, ein uni- oder bipolares Impedanz- oder Leitfähigkeitssignal durch Messen eines Impedanz- oder Leitfähigkeitswertes zwischen einer Neutralelektrode (26) und einer Messelektrode (16) oder zwischen zwei Messelektroden zu erzeugen sowie eine Auswerteeinheit (38) angeordnet sind,

wobei die Auswerteeinheit mit der Impedanz- oder Leitfähigkeitsmesseinheit und dem Aktivitätssensor verbunden und zum Auswerten des von der Impedanz- oder Leitfähigkeitsmesseinheit erzeugten Impedanz- oder Leitfähigkeitssignals und des jeweils zeitlich zugeordneten Aktivitätspegefsignals und zum Erzeugen und Ausgeben eines Kontraktilitätssignals derart ausgebildet ist, dass das Kontraktilitätssignal aus dem Impedanz- oder Leitfähigkeitssignal sowie dem Aktivitätspegelsignal abgeleitet ist und den jeweiligen kontraktilen Zustand eines Herzens zugeordnet zu einem Aktivitätspegelsignalwert wiederspiegelt und das Elektrotherapiegerät ausgebildet ist, jeweils ein zeitabgetastetes (gesampeltes) Impedanz- oder Leitfähigkeitsverlaufsignal und ein zeitabgetastetes Aktivitätspegelsignal zeitlich dem jeweiligen Impedanz- oder Leitfähigkejtsvertaufsignal zugeordnet zu erzeugen,

**dadurch gekennzeichnet, dass** die Auswerteeinheit ausgebildet ist, einen Referenzsignalverlauf aus wenigstens einem Impedanz- oder Leitfähigkeitssignalverlauf in Abhängigkeit von gleichzeitig aufgenommenen Aktivitätspegelsignalwerten zu bilden und

die Auswerteeinheit ausgebildet ist, den Kontraktilitätssignal-Verlauf mit einem jeweils zeitlich zugeordneten Aktivitätspegelsignalwert oder einer Aktivitätspegelsignalwertdifferenz zu normieren.

2. Elektrotherapiegerät nach Anspruch 1, **dadurch gekennzeichnet, dass** das Gehäuse eine Neutralelektrode umfasst und die Impedanz- oder Leitfähigkeitsmesseinheit ausgebildet ist, ein unipolares Impedanz- oder Leitfähigkeitssignal durch Messen eines Impedanz- oder Leitfähigkeitswertes zwischen einer Neutralelektrode und einer Messelektrode zu erzeugen und dass die Auswerteeinheit ausgebildet ist, das Kontraktilitätssignal aus dem unipolaren Impedanz- oder Leitfähigkeitssignal abzuleiten.

3. Elektrotherapiegerät nach Anspruch 1, **dadurch gekennzeichnet, dass** die Auswerteeinheit ausgebildet ist, das Kontraktilitätssignal als Kontraktilitätsdifferenzsignal in Abhängigkeit von dem Impedanz- oder Leitfähigkeitsverlaufssignal durch Bilden einer Differenz zwischen zwei in unterschiedlichen Zeiträumen aufgenommenen Impedanz- oder Leitfähigkeitsverlaufssignalen derart ausgebildet ist, dass ein jeweiliger Kontraktilitätsdifferenzwert des Kontraktilitätsdifferenzsignals von einer Fläche abhängt, die die zwei zu unterschiedlichen Zeiträumen erfassten Impedanz- bzw. Leitfähigkeitsverlaufssignale zwischen sich einschließen.

4. Elektrotherapiegerät nach Anspruch 5, **dadurch gekennzeichnet, dass** die Auswerteeinheit ausgebildet ist, einem jeweiligen Kontraktilitätsdifferenzwert einen Aktivitätspegelsignalwert zuzuordnen, der für wenigstens einen Zeitraum gilt, der wenigstens einem der Impedanz- oder Leitfähigkeitsverlaufssignale zugeordnet ist.

5. Elektrotherapiegerät nach Anspruch 1, **dadurch gekennzeichnet, dass** die Auswerteeinheit ausgebildet ist, den Referenzsignalverlauf durch Mittelwertbildung aus mehreren Impedanz- oder Leitfähigkeitssignalverläufen zu bilden.

6. Elektrotherapiegerät nach Anspruch 5, **dadurch gekennzeichnet, dass** die Auswerteeinheit ausgebildet ist, zum Bilden des Referenzsignalverlaufs durch Mittelwertbildung nur solche Impedanz- oder Leitfähigkeitssignalverläufe zu berücksichtigen, denen jeweils ein Aktivitätspegelsignalwert unterhalb eines vorgegebenen Grenzaktivitätspegelwertes zeitlich zugeordnet ist.

7. Elektrotherapiegerät nach einem der Ansprüche 1 bis 6, **dadurch gekennzeichnet, dass** das Elektrotherapiegerät derart ausgebildet ist, das ein jeweiliger Zeitraum, über die ein jeweiliges Impedanz- oder Leitfähigkeitsverlaufssignal aufzunehmen oder zu bilden ist, die Dauer von wenigstens einem definierten Teil eines Herzzyklusses oder eines ganzen Herzzyklusses oder einer ganzzahligen Anzahl von wenigen Herzzyklen.

8. Elektrotherapiegerät nach einem der Ansprüche 1 bis 7, **dadurch gekennzeichnet, dass** die Impedanz- oder Leitfähigkeitsmesseinheit einen Eingangsverstärker (34) mit einer automatischen Eingangsverstärkungssteuerung (ACG) aufweist, so dass die jeweilige Verstärkung eines Ausgangssignal eines Impedanz- oder Leitfähigkeitssensors der Impedanz- oder Leitfähigkeitsmesseinheit möglichst optional ist, wobei die Impedanz- oder Leitfähigkeitsmesseinheit ausgebildet ist, ein Ausgangssignal des Eingangsverstärkers mit einem jeweils zugehörigen Verstärkungsfaktor zu skalieren.

9. Elektrotherapiegerät nach einem der Ansprüche 1 bis 8, **dadurch gekennzeichnet, dass** in dem Gehäuse eine Therapiegerätesteuereinheit (44) vorgesehen ist, die mit der Auswerteeinheit verbunden ausgebildet ist, und wenigstens einen Therapieparameter wie uni- oder biventrikuläre Stimulation, interventrikuläre Verzögerungszeit, atrio-ventrikuläre Verzögerungszeit in Abhängigkeit eines jeweiligen Kontraktilitätssignals oder eines Kontraktilitätsdifferenzsignals einzustellen.

10. Elektrotherapiegerät nach Anspruch 9, **dadurch gekennzeichnet, dass** die Therapiegerätesteuereinheit ausgebildet ist, den jeweiligen Therapieparameter derart einzustellen, dass das Kontraktilitätssignal oder das Kontraktilitätsdifferenzsignal eine maximale Kontraktilität anzeigt.

11. Elektrotherapiegerät nach einem der Ansprüche 1 bis 10, **dadurch gekennzeichnet, dass** in dem Gehäuse ein Datenspeicher (42) für das Kontraktilitätssignal und vorzugsweise zusätzlich für das Aktivitätspegelsignal vorgesehen ist.

12. Elektrotherapiegerät nach Anspruch 11, **dadurch gekennzeichnet, dass** der Datenspeicher mehrere Speicherbereiche für einen Referenzsignalverlauf und für wenigstens einen aktuellen Impedanz- oder Leitfähigkeitssignalverlauf sowie für einen jeweils zugeordneten Aktivitätspegelsignalverlauf aufweist.

13. Elektrotherapiegerät nach Anspruch 9 oder 10 und Anspruch 11 oder 12, **dadurch gekennzeichnet, dass** der Datenspeicher mit der Therapiesteuereinheit verbunden ist.

14. Elektrotherapiegerät nach einem der Ansprüche 11 bis 13, **dadurch gekennzeichnet, dass** der Datenspeicher mit der Auswerteeinheit verbunden ist.

15. Elektrotherapiegerät nach einem der Ansprüche 9 bis 14, **dadurch gekennzeichnet, dass** in dem Gehäuse eine Datentelemetrieeinheit (46) angeordnet ist, die mit der Therapiesteuereinheit oder dem Datenspeicher oder beiden verbunden ist.

**Claims**

1. An implantable electrotherapy device, in particular a cardiac pacemaker, defibrillator, or the like having a housing (26), which
   has an electrode line terminal for connecting at least one electrode line to be placed intracardially, which has at least one measuring electrode for recording an intracardial impedance signal,
   an activity sensor (40), which is implemented to generate an activity level signal, whose particular activity level signal values are a function of a physical activity of a patient in the implanted state of the therapy device,
   an impedance or conductivity measuring unit (30), which is implemented to generate a unipolar or bipolar impedance or conductivity signal by measuring an impedance or conductivity value between a neutral electrode (96) and a measuring electrode (16) or between two measuring electrodes, and
   an analysis unit (38) being situated in the housing, the analysis unit being connected to the impedance or conductivity measuring unit and the activity sensor and being implemented to analyze the impedance or conductivity signal generated by the impedance or conductivity measuring unit and the particular chronologically assigned activity level signal and to generate and output a contractility signal in such a way that the contractility signal is derived from the impedance or conductivity signal and the activity level signal and reflects the particular contractile state of a heart assigned to an activity level signal value and the electrotherapy device is implemented to generate a time-sampled impedance or conductivity curve signal and a time-sampled activity level signal assigned chronologically to the particular impedance or conductivity curve signal,
   **characterized in that** the analysis unit is implemented to calculate a reference signal curve from at least one impedance or conductivity signal curve as a function of simultaneously recorded activity level signal values and
   the analysis unit is implemented to scale the contractility signal curve using a particular chronologically assigned activity level signal value or an activity level signal value difference.

2. The electrotherapy device according to Claim 1, **characterized in that** the housing comprises a neutral electrode and the impedance or conductivity measuring unit is implemented to generate a unipolar impedance or conductivity signal by measuring an impedance or conductivity value between a neutral electrode and a measuring electrode, and the analysis unit is implemented to derive the contractility signal from the unipolar impedance or conductivity signal.

3. The electrotherapy device according to Claim 1, **characterized in that** the analysis unit is implemented to generate the contractility signal as a contractility differential signal as a function of the impedance or conductivity curve signal by calculating the difference between two impedance or conductivity curve signals recorded in different periods of time in such a way that a particular contractility differential value of the contractility differential signal is a function of an area which two impedance and/or conductivity curve signals acquired at different periods of time enclose between themselves.

4. The electrotherapy device according to Claim 3, **characterized in that** the analysis unit is implemented to assign an activity level signal value, which applies for at least one period of time which is assigned to at least one of the impedance or conductivity curve signals, to a particular contractility differential value.

5. The electrotherapy device according to Claim 1, **characterized in that** the analysis unit is implemented to calculate the reference signal curve by averaging from multiple impedance or conductivity signal curves.

6. The electrotherapy device according to Claim 5, **characterized in that** the analysis unit is implemented to calculate the reference signal curve by calculating only those impedance or conductivity signal curves to which an activity level signal value below a predefined limiting activity level value is chronologically assigned in each case.

7. The electrotherapy device according to one of Claims 1 through 6, **characterized in that** the electrotherapy device is implemented in such a way that a particular period of time over which a particular impedance or conductivity curve signal is to be recorded or calculated has the duration of at least one defined part of a cardiac cycle or an entire cardiac cycle or an integral number of a few cardiac cycles.

8. The electrotherapy device according to one of Claims 1 through 7, **characterized in that** the impedance or conductivity measuring unit has an input amplifier (34) having an automatic input gain control (AGC), so that the particular amplification of an output signal of an impedance or conductivity sensor of the impedance or conductivity measuring unit is as optimal as possible, the impedance or conductivity measuring unit being implemented to scale an output signal of the input amplifier using a particular assigned amplification factor.

9. The electrotherapy device according to one of Claims 1 through 8, **characterized in that** a therapy device control unit (44) is provided in the housing, which is connected to the analysis unit and is implemented to set at least one therapy parameter, such as univentricular or biventricular stimulation, interventricular delay time, or atrial-ventricular delay time as a function of a particular contractility signal or a contractility differential signal.

10. The electrotherapy device according to Claim 9, **characterized in that** the therapy device control unit is implemented to set the particular therapy parameter in such a way that the contractility signal or the contractility differential signal indicates a maximum contractility.

11. The electrotherapy device according to one of Claims 1 through 10, **characterized in that** a data memory (42) for the contractility signal and preferably also for the activity level signal is provided in the housing.

12. The electrotherapy device according to Claim 11, **characterized in that** the data memory has multiple memory areas for a reference signal curve and for at least one current impedance or conductivity signal curve as well as for a particular assigned activity level signal curve.

13. The electrotherapy device according to Claim 9 or 10 and Claim 11 or 12, **characterized in that** the data memory is connected to the therapy control unit.

14. The electrotherapy device according to one of Claims 11 through 13, **characterized in that** the data memory is connected to the analysis unit.

15. The electrotherapy device according to one of Claims 9 through 14, **characterized in that** a data telemetry unit (46), which is connected to the therapy control unit or the data memory or both, is situated in the housing.

**Revendications**

1. Appareil d'électrothérapie implantable, en particulier pacemaker, défibrillateur ou similaire, comprenant un boîtier (26), qui
présente un branchement de ligne d'électrode pour le branchement d'au moins une ligne d'électrode à placer de façon intracardiaque, qui comporte au moins une électrode de mesure pour l'enregistrement d'un signal d'impédance intracardiaque,
un capteur d'activité (40), qui est conçu pour générer un signal de niveau d'activité, dont les valeurs respectives de signal d'activité dépendent d'une activité physique d'un patient lorsque l'appareil de thérapie est dans l'état implanté,
une unité de mesure d'impédance ou de conductibilité (30), qui est conçue pour générer un signal d'impédance ou de conductibilité unipolaire ou bipolaire par la mesure d'une valeur d'impédance ou de conductibilité entre une électrode neutre (26) et une électrode de mesure (16) ou entre deux électrodes de mesure, et une unité d'analyse (38) étant disposées dans le boîtier,
l'unité d'analyse étant reliée à l'unité de mesure d'impédance ou de conductibilité et au capteur d'activité et étant réalisée pour analyser le signal d'impédance ou de conductibilité généré par l'unité de mesure d'impédance ou de conductibilité et le signal de niveau d'activité respectivement attribué dans le temps et pour générer et délivrer un signal de contractilité de telle sorte que le signal de contractilité est déduit du signal d'impédance ou de conductibilité et du signal de niveau d'activité et reflète l'état contractile respectif d'un coeur attribué à une valeur de signal de niveau d'activité et l'appareil d'électrothérapie étant réalisé pour générer à chaque fois un signal de courbe d'impédance ou de conductibilité balayé dans le temps (échantillonné) et un signal de niveau d'activité balayé dans le temps avec une attribution dans le temps au signal respectif de courbe d'impédance ou de conductibilité,
**caractérisé en ce que** l'unité d'analyse est conçue pour former une courbe de signal de référence à partir d'au moins une courbe de signal d'impédance ou de signal de conductibilité en fonction de valeurs de signal de niveau d'activité enregistrées en même temps et
l'unité d'analyse est conçue pour normaliser la courbe de signal de contractilité avec une valeur de signal de niveau d'activité respectivement attribuée dans le temps ou une différence de valeur de signal de niveau d'activité.

2. Appareil d'électrothérapie selon la revendication 1, **caractérisé en ce que** le boîtier comporte une électrode neutre et l'unité de mesure de l'impédance ou de conductibilité est réalisé pour générer un signal d'impédance ou de conductibilité unipolaire par la mesure d'une valeur d'impédance ou de conductibilité entre une électrode neutre et une électrode de mesure et **en ce que** l'unité d'analyse est réalisé pour déduire le signal de contractilité du signal d'impédance ou de conductibilité unipolaire.

3. Appareil d'électrothérapie selon la revendication 1, **caractérisé en ce que** l'unité d'analyse est réalisée, le signal de contractilité en tant que signal de différence de contractilité est réalisé en fonction du signal de courbe d'impédance ou de conductibilité par la formation d'une différence entre deux signaux de courbe d'impédance ou de conductibilité enregistrés à différentes périodes de telle sorte qu'une valeur respective de différence de contractilité du signal de différence de contractilité dépend d'une surface qui renferme les deux signaux de courbe d'impédance ou de conductivité enregistrés à différentes périodes.

4. Appareil d'électrothérapie selon la revendication 3, **caractérisé en ce que** l'unité d'analyse est réalisée pour attribuer à une valeur respective de différence de contractilité une valeur de signal de niveau d'activité qui vaut pour au moins une période qui est attribuée à au moins l'un des signaux de courbe d'impédance ou de conductibilité.

5. Appareil d'électrothérapie selon la revendication 1, **caractérisé en ce que** l'unité d'analyse est conçue pour former la courbe de signal de référence par la formation de valeur moyenne à partir de plusieurs courbes de signal d'impédance ou de conductibilité.

6. Appareil d'électrothérapie selon la revendication 5, **caractérisé en ce que** l'unité d'analyse est réalisée pour tenir compte, pour la formation de la courbe de signal de référence par la formation de moyenne, uniquement de courbes de signal d'impédance ou de conductibilité, à chacune desquelles une valeur de signal de niveau d'activité est attribuée dans le temps au-dessous d'une valeur prédéfinie de niveau d'activité limite.

7. Appareil d'électrothérapie selon l'une quelconque des revendications 1 à 6, **caractérisé en ce que** l'appareil d'électrothérapie est réalisé de telle sorte qu'une période respective, pendant laquelle un signal respectif de courbe d'impédance ou de conductibilité doit être enregistré ou formé, est la durée d'au moins une partie définie d'un cycle cardiaque ou d'un cycle cardiaque entier ou d'un nombre pair de quelques cycles cardiaques.

8. Appareil d'électrothérapie selon l'une quelconque des revendications 1 à 7, **caractérisé en ce que** l'unité de mesure d'impédance ou de conductibilité présente un amplificateur d'entrée (34) avec une commande automatique d'amplification d'entrée (ACG), de sorte que l'amplification respective d'un signal de sortie d'un capteur d'impédance ou de conductibilité de l'unité de mesure d'impédance ou de conductibilité est si possible optionnelle, l'unité de mesure d'impédance ou de conductibilité étant réalisée pour graduer un signal de sortie de l'amplificateur d'entrée avec un facteur d'amplification respectif.

9. Appareil d'électrothérapie selon l'une quelconque des revendications 1 à 8, **caractérisé en ce qu'**il est prévu dans le boîtier une unité de commande d'appareil de thérapie (44) qui est reliée à l'unité d'analyse et est conçue pour régler au moins un paramètre de thérapie comme une stimulation univentriculaire ou biventriculaire, une temporisation interventriculaire, une temporisation atrioventriculaire en fonction d'un signal respectif de contractilité ou d'un signal de différence de contractilité.

10. Appareil d'électrothérapie selon la revendication 9, **caractérisé en ce que** l'unité de commande d'appareil de thérapie est conçue pour régler le paramètre de thérapie respectif de telle sorte que le signal de contractilité ou le signal de différence de contractilité affichent une contractilité maximale.

**11.** Appareil d'électrothérapie selon l'une quelconque des revendications 1 à 10, **caractérisé en ce qu'**il est prévu dans le boîtier une mémoire de données (42) pour le signal de contractilité et de préférence en supplément pour le signal de niveau d'activité.

**12.** Appareil d'électrothérapie selon la revendication 11, **caractérisé en ce que** la mémoire de données présente plusieurs zones de mémoire pour une courbe de signal de référence et pour au moins une courbe actuelle de signal d'impédance ou de conductibilité ainsi que pour une courbe respectivement attribuée de signal de niveau d'activité.

**13.** Appareil d'électrothérapie selon la revendication 9 ou 10 et la revendication 11 ou 12, **caractérisé en ce que** la mémoire de données est reliée à l'unité de commande de thérapie.

**14.** Appareil d'électrothérapie selon l'une quelconque des revendications 11 à 13, **caractérisé en ce que** la mémoire de données est reliée à l'unité d'analyse.

**15.** Appareil d'électrothérapie selon l'une quelconque des revendications 9 à 14, **caractérisé en ce que** dans le boîtier est disposée une unité de télémétrie de données (46) qui est reliée à l'unité de commande de thérapie ou à la mémoire de données ou aux deux.

FIG. 1

Potential

FIG. 2

# FIG. 3

Differenzfläche DA

belastet

unbelastet/Ruhezustand

Rest

Load

Differential Area

Impedanz (normiert)

Zeit nach ventr. Stimulus (ms)

# FIG. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4674518 A
- US 5417717 A

- EP 1062974 A